# EUROPEAN PATENT SPECIFICATION

(11) **EP 2 200 759 B1**
(45) Date of publication and mention of the grant of the patent: **20.06.2012**
(21) Application number: 08801360.2
(22) Date of filing: 15.09.2008
(51) Int. Cl.: B08B 3/00, B08B 13/00

(54) **EQUIPMENT IN COMBINATION WITH HOSPITAL- AND CARE FURNITURE AND METHOD OF CONTROLLING THE CLEANING AND SERVICING OF THE FURNITURE**
AUSRÜSTUNG IN KOMBINATION MIT KRANKENHAUS- UND PFLEGEEINRICHTUNGEN UND VERFAHREN UM DIE REINIGUNG UND WARTUNG DER EINRICHTUNG ZU ÜBERWACHEN
EQUIPEMENT EN COMBINAISON AVEC DU MOBILIER D'HÔPITAL ET DE DE SOINS ET MÉTHODE DE SURVEILLANCE DU NETTOYAGE ET DE L'ENTRETIEN DE CE MOBILIER

(30) Priority: 13.09.2007 DK 200701323
(43) Date of publication of application: 30.06.2010
(73) Proprietor: Linak A/S, 6430 Nordborg (DK)
(72) Inventor: JENSEN, Peter Brøndum, DK- 6400 Sønderborg (DK)
(74) Representative: Pedersen, Soeren Skovgaard
(86) International application number: PCT/DK2008/000325
(87) International publication number: WO 2009/033487

(56) References cited:
- EP-A- 0 498 111
- DE-A1-102006 008 723
- US-A1- 2006 223 731

## Description

The invention relates to an equipment in combination with a piece of hospital and care furniture, e.g. a hospital or care bed, a patient lifter, a treatment table, a chair as stated in the preamble of claim 1. Further, the invention relates to a method for cleaning of hospital- and care furniture and a method for servicing of hospital and care furniture based on the cleaning of these, using such an equipment as defined in claims 22 and 23.

Document EP-0498111 describes a hospital and care furniture comprising an activator for adjusting the furniture, a control unit and a control comprising a microprocessor.

In hospitals and nursing homes good hygiene is a cogent necessity, as it can have fatal consequences especially if weakened persons are infected with dangerous bacteria or viruses. Thus, great precautions are taken to ensure that everything is carefully disinfected and cleaned.

Typically the bed, i.e. bed frame with framework and system for adjusting the bed, is transported to a central cleaning department and driven through a washer system, which is constructed specially for cleaning articles of hospital- and care furniture. Such a washer system is e.g. known from FR 2 744 041 to Athen. Cleaning of mattresses can be performed in a washer system specially constructed for cleaning mattresses, as known from DE 4 108 536 to Sauter Moller GMBH. Pillows, duvets and bed linen are treated in a separate washer system.

It is one thing to be in possession of a washer system for cleaning the bed and another to ensure that the bed is also cleaned. This especially applies, when the bed is used by a long-term hospitalized patient. It is also important that a bed is cleaned before it is occupied by another patient. Lack of cleaning of a bed would be particularly unfortunate if the patient leaving the bed has had a contagious illness or has contaminated the bed with bodily fluids or other substances capable of transmitting infections or providing a breeding ground for bacteria.

The purpose of the invention is to provide a solution to the outlined problem, which is to ensure cleaning of hospitals- and care beds.

This is achieved according to the invention with the solution as stated in claims 1, 22 and 23.

Here, it is stated that the equipment comprises a signaling device for signaling when the piece of furniture has been cleaned, a receiver for receiving the signal from the signaling device, a timer and/or counter unit, which based on the signal starts a process to determine when the article of furniture again needs cleaning and an indicator e.g. arranged in connection with the piece of furniture, which shows when the piece of furniture again needs cleaning.

The equipment is characteristic by having an initiation function, which can be activated when the article of furniture has been cleaned. When this initiation function is activated, a timer starts which after the expiration of the preset period of time, gives a signal, indicating the requirement for cleaning of the piece of furniture.

It would be practical to be able to adjust the period of time which elapses before the indication of "cleaning required" occurs, either continuously in hours, days or weeks so that any wish for indication of the need for cleaning can be met. Three steps could e.g. be incorporated, where an operation key could alternate between intervals of one, two or three weeks. In order to avoid unintended operation and thereby risk lack of cleaning of the piece of furniture, it could be implemented that the key should be pressed for a given time, before the adjustment is achievable.

Further, the equipment can be supplied with input about the department in which the piece of furniture is located, and if it concerns a bed, then what kind of patients are using it, in order thus to be able to vary the period of time that has elapsed before the indication of "cleaning required" is triggered, partly depending on the vulnerability of the patients but also on how immense a source of infection that patient can be to the surroundings.

Simultaneously with the timer process, a parallel process is started, where a counter registers the number of activations of the hand control. When the counter exceeds a preset number, the requirement for cleaning of the bed is indicated.

A particular immense source of infection can be keyboards which from time to time are touched by both patients and nursing staff. By counting the number of activations of the control unit, an overview of its cleaning status can be created. Whether the required cleaning is limited to the control unit as an extraordinary cleaning task or other units of the bed or maybe the surroundings of the bed should be decided by the nursing staff. In case more control units are attached to the bed, the indication of "cleaning required" can be triggered as a result of numerous scenarios, e.g. when the amount of activations exceeds a preset threshold, or when one of the control units exceeds a preset threshold.

The indication that cleaning is required will therefore be triggered either by an expiration of time or the frequency of operations, whichever of the two occurrences expires first.

The equipment is initiated by operating a switch on the control panel when the piece of furniture has been cleaned. In case the cleaning takes place in a washer system, the initiation can be performed automatically in that a switch on the piece of furniture is activated when the article of furniture is transported out of the washer system. The switch-function can be constructed with a conventional switch or with a sensor, e.g. a magnet-based sensor activated by a magnet. When the system is initiated, a timer e.g. a counter or a clock starts to increment itself with an appropriate interval.

In a special embodiment, the equipment is furnished with a thermo sensor which gives a signal, when the temperature exceeds a threshold value. In that way, it is possible to automatically register that the piece of furniture is undergoing cleaning in a washer system. The signal can be used for automatically resetting the timer/counter in the equipment so that a new timer/counter period can elapse before the equipment again raises the alarm. The signal from the thermo sensor is practically constructed with a hysteresis so that the signal does not disappear before the temperature has dropped considerably, usually combined with a period of time which also have to expire. This prevents the signal from fluctuating and is important in connection with the wish for registering the number of cleanings of the article of furniture.

As a special function, the control panel can be equipped with a function, where the cleaning status manually can be set to "cleaning required". This ensures that the piece of furniture, e.g. a bed, will be cleaned before the next patient occupies it, or in case the bed has been extraordinarily soiled or contaminated with viruses or bacteria.

When the counter reaches the preset values, the indications showing the cleaning status of the piece of furniture are altered. In a simple embodiment, the indicators can constitute a green indicator, e.g. in the form of a light-emitting diode, for "status ok" and a red indicator for "cleaning required", but indicators for conditions in-between the two mentioned extremes can naturally also be incorporated and the choice of color of the different indicators is naturally also open. It is also possible to use a display, for graphically, alphanumerically or numerically showing the cleaning status of the piece of furniture. By observing the indicators, the staff can decide whether a piece of furniture needs cleaning.

The adjustment of a piece of hospital or care furniture is normally operated by means of a hand control. The patient typically has his own control unit with a limited number of functions, whereas the staff has access to a control unit with more functions. The initiation function and the indicators, being a result of the invention, can therefore be implemented in the control unit dedicated to the nursing staff.

It is, however, not excluded that an indication for the cleaning status of the piece of furniture is located elsewhere on the piece of furniture to ensure a fast and clear overview of whether cleaning is needed. It will also be possible to communicate the status on to a central operator terminal located some distance away from the article of furniture.

Especially expedient, the indication can vary depending on which situation has triggered it, so that a triggering of a "cleaning required" indication based on both time and use wise criteria or a manual adjustment differs from each other. A distinction in the indication can be achieved by introducing separate indicators for the individual situations having triggered the requirement for cleaning. If only one indicator is preferred, this can change its color or blink at different frequencies depending on what has triggered the requirement for cleaning of the piece of furniture, and in that way indicate the extent of the need for cleaning.

The system could be extended in such a way that the requirement for cleaning can be adjusted in levels, which reflects the need for execution of different cleaning routines distributed between the nursing staff. When it comes to a bed, it can be cleaning of the bed frame with framework and adjustment mechanism, cleaning of a mattress, replacement of duvet, pillow or bed linen or extraordinary cleaning of other parts of the bed, like e.g. control units. It would then make sense to construct the equipment so that individual indications are given when these cleaning routines should be performed.

The invention can either be realized as a separate unit or an expansion of the existing control managing the adjustment of the article of furniture.

The equipment is supplied with power from the mains power supply, possibly via the control. As it is not desired that the equipment is reset or loses information if it loses its supply, it is expedient to have an extra backup in the form of at least one battery. It is most ideally if the equipment in the microprocessor control is furnished with a memory circuit for retaining the set-up and data even if the power supply is completely cut off. As the timer interval between cleanings is long, the microprocessor is designed to hibernate and at regular intervals wake up to update the status. This would result in minimum energy consumption. At the same time, the energy consumption can be minimized if the indication panel is allowed to be dimmed or turned off. In order to give the staff the impression that the equipment is still in operation, one of the diodes can at regular intervals be brought to a brief blink. The dimming function can be constructed in such a way that the staff by pressing the keys can dim or turn off the indication panel and with a brief press can read the status on the indication panel before it, after a short period of time, again automatically will dim or turn off. Ideally, the equipment will itself minimize the power consumption when the supply to the mains is cut off, so that the energy of a backup battery supply is drained as little as possible. By furnishing the equipment with a real-time clock or providing access to such a clock in the network, it will be possible to build a routine, which based on the last occurrence can reconstruct and update timer data based on the incorporated routines and formulas.

The cleaning of the article of furniture is a necessary provision, which however has the disadvantage that it burdens the mechanical state of the piece of furniture. For instance, it is certain that the lubricant in time will disappear when the piece of furniture is washed in a washer system. For that reason, the piece of furniture should at regular intervals be inspected and mechanical movable parts additionally treated and lubricated. By introducing an extra counter in the equipment, which counts the number of performed cleanings, the equipment would further be able to indicate a need for service after a predefined number of cleanings. The required service can then be performed after which the indicator can be reset. In order to avoid unintended resetting of the service indication, a combined signal can be used when resetting, consisting of a pressing of the keys at the same time as a Hall sensor is activated by moving a magnet in its vicinity. The Hall sensor is connected to the microprocessor which processes the signals from the keys and the Hall sensor so that the microprocessor can perform the resetting of the counter. The communication between the microprocessor and other units in connection with the piece of furniture also involves the ability to externally control and reset the counter for indication of service.

The position of the Hall-sensor against one of the sides in the housing should then either be known or indicated on the housing.

The indication of the requirement for service can be visually presented on a panel of light-emitting diodes, where the simplest embodiment is a light-emitting diode, which is switched-on when service is required. The panel can be expanded so that more light-emitting diodes show the status of the process, so that it beforehand can be chosen to perform servicing if it fits the time table better. In more advanced solutions, displays can be used, e.g. seven segment digit display or graphic displays with text and figures. The indicators can be located in a housing which subsequently can be mounted on the piece of furniture or in a control unit for adjusting the piece of furniture. It is also possible to communicate the status on to a central operator terminal located at a distance from the article of furniture.

The requirement for service after cleaning is reinforced when using stronger cleaning agents. It is not uncommon that highly acid or alkaline cleaning agents are used, which can ruin seals in the electric actuators or housings for electric controls, so that these are no longer waterproof. At the same time lubricants in the electric drives or in the mechanical connections on the piece of furniture are degraded or washed away. It will therefore be an advantage if the equipment is also furnished with an instrument for measuring the pH value during washing and give a signal for accelerating servicing of the article of furniture if aggressive cleaning agents are used for the cleaning. The measuring instrument can be located outside the housing and be connected via a cable or be positioned in the housing. The signal from the pH sensor as input for the counter for the number of cleanings means that the number of cleanings to be performed between servicing can vary from a determine formula or table, depending on intensity of the cleaning of the piece of furniture.

For communication between the equipment for use in connection with cleaning of articles of hospital and care furniture and the control, which manages the adjustment of the bed, the communication system "Openbus" according to WO2007/057014 to Linak is used. Openbus describes a standard for communication between the various units attached to the bed, this being hand control(s), the individual actuator, etc. It is therefore possible to communicate various parameters between the control and the individual Openbus-connected unit. The control can via a gateway be connected to networks in the hospital which can comprise a central monitoring post, operated by the nursing staff and/or staff in charge of maintenance and cleaning. With "Openbus" is provided several possibilities for communication of status on the piece of furniture including duplex communication directly with the equipment for use in connection with cleaning of articles of hospital and care furniture. It will thus be possible to transfer the indication "cleaning required" or "servicing required" with various parameters as to what needs cleaning or servicing, directly to the central monitoring. The sorting of the indication can take place so that a request for cleaning is sent directly to the cleaning staff or the hospital porter, who is in charge of collecting the piece of furniture and bringing it to cleaning. When it comes to external companies which take care of maintenance, an indication of service or repair could e.g. be sent via the internet from the monitoring post. If the monitoring post is connected to the database containing data about registered patients, it will from the monitoring post, based on the hygienic condition of the patient, be possible to program the parameters of the cleaning indication system, so that a request for intensified hygiene e.g. will trigger an indication of "cleaning required" earlier than, what is otherwise standard for the cleaning indication system. When a patient is discharged, it will be possible from the monitoring post manually or automatically to externally control the equipment for use in connection with cleaning of hospital- and care furniture to show "cleaning required" and/or "servicing required", and thus ensuring that the piece of furniture will be cleaned and serviced before the next patient occupies it.

The invention also relates to a method for use in connection with cleaning of hospital and care furniture, including an initiation or reset state, an operating condition with indication of the status and an alarm condition, where the initiation state, is carried out manually or automatically with a sensor, preferably a thermo sensor, resets a timer, which goes through a period of time before the next cleaning and resetting of a counter, which counts the number of operations of a control unit, such as a hand control, and at the same time updates the status on an indication panel and where the operating condition maintains and increments the timer in the process of a preset period of time and indicates the status of the process on the indication panel, and at the same time maintains the counter, which counts the number of operations of the control unit, and where the alarm condition occurs when the preset time of the timer expires or when the counter, which counts the number of operations of the control unit exceeds a determined number, where the alarm condition is indicated on the indication panel.

Further, the invention comprises a method for use in connection with servicing of hospital and care furniture based on the cleaning of these, which includes a initiation or reset state, an operation condition with indication of status and an alarm condition, where the initiation state is carried out manually by activating a sensor, preferably a Hall-sensor with a magnet, and brings about a resetting of a counter, which counts the number of cleanings and at the same time updates the status on an indication panel, and where the operating condition maintains the counter, which counts the number of cleanings of the piece of furniture and as input also has a sensor, preferably a pH sensor, which measures the aggressiveness of the cleaning agent and manipulates the counter with a factor corresponding to the extra need for service and where the alarm condition occurs when the counter, which counts the number of cleanings of the piece of furniture, exceeds a determined number, where the alarm condition is indicated on the indication panel.

The equipment according to the invention will be described more fully below with reference to the accompanying drawing, in which
Fig. 1, shows a hospital bed in a washer system,
Fig. 2, shows a hospital bed having the equipment mounted thereon,
Fig. 3, shows a flow chart of the equipment, and
Fig. 4, shows a housing for the equipment.

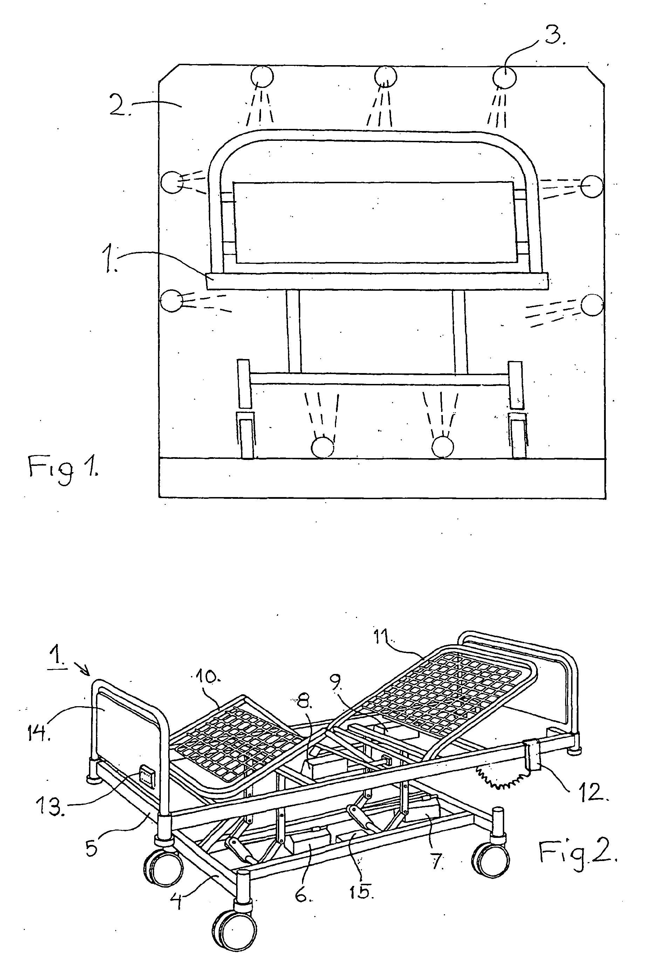

Fig. 1 of the drawing shows a hospital bed 1 during cleaning in a washer system 2 specially constructed for cleaning hospital beds 1. The washer system 2 is constructed with a number of jets 3 through which the cleaning agent and water is injected into the chamber of the washer system and thus efficiently cleaning the hospital bed.

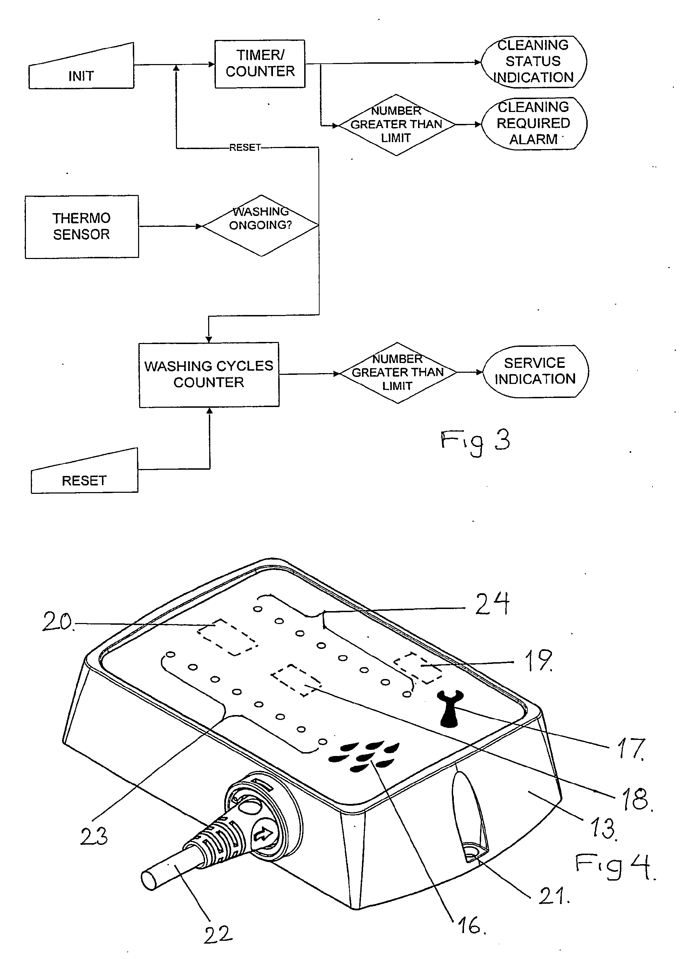

The hospital bed 1 is further shown in Fig. 2, where it is apparent that it is constructed with a lower frame 4 and a an upper frame 5, which are height-adjustable in proportion to each other by activation of the electric linear actuators 6, 7. The electric linear actuators 8, 9 serve to adjust the support surfaces 10,11. The adjustment is carried out by operating a hand control 12. The equipment 13 is shown as a separate unit mounted on a bed end 14 at the foot end of the bed. The equipment incorporated in its own housing is incidentally shown in Fig. 4. Even though the equipment here is shown in its own housing, there is nothing to prevent the equipment from being integrated in the control box 15 for the actuators comprising the control. The flow chart in Fig. 3 shows that the system is initiated when the bed has been cleaned, after which a timer process and/or counter process is started. On an indication panel, the status of the equipment is shown so that it becomes apparent,how much time has elapsed so far, and is left until the bed needs cleaning. A counter is started parallel to the process and counts the number of operations of the actuator system. When timer and/or counter reach a threshold value, the indication that the bed needs cleaning is started. The system can be reset either by operating the keys 16, 17 of the panel in a certain sequence or by means of a switch on the bed, which can be activated manually. It could e.g. be a key operated switch or alternatively a magnetic switch in connection with the bed, which can be activated by means of a magnet located in the washer system. In a special embodiment, the equipment is furnished with a thermo sensor 18 which gives a signal when the temperature exceeds a threshold value. In that way, it is possible to automatically register that the bed is undergoing cleaning in a washer system 2. The signal can be used for automatically resetting the timer/counter so that a new timer/counter period can elapse before the equipment again raises the alarm. The signal from the thermo sensor 18 is practically constructed with a hysteresis so that the signal does not disappear before the temperature has dropped considerably, usually combined with a period of time which also must expire. This prevents the signal from fluctuating and is important in connection with the wish for registering the number of cleanings of the article of furniture. The number of performed cleanings can be used statistically, but also to estimate when the bed should be serviced. It is especially practical to establish a counter here, which registers the number of performed cleanings, so that a number of cleanings triggers an indication that servicing of the bed is required. Such an indication should be able to be reset, but only by authorized staff. For that reason a magnetic sensor 19 is built into the housing, so that it is possible to place a magnet outside the housing in its vicinity to be able to perform a reset of the indication. Practically, the reset function would be able to discontinue a simultaneous key combination, so that unintended operation is avoided. As the service interval is not only determined by the number of cleanings of the bed, but also of the strength of the cleaning agent, a pH sensor 20 is introduced, to provide possibility for accelerating servicing if this is required. A formula or table in the microprocessor of the control can be constructed so that the change in the service interval precisely can be determined in the light of how aggressive cleaning agents are used to clean the bed.

As it appears from Fig. 4 of the drawing the equipment is built-in in its own housing 13 and appears in this embodiment as a separate unit. On the housing there is a section 21 with holes for attachment for assembly of the housing on the bed unit, typically on the bed frame 5 or the bed end 14. The housing is supplied with power via the cable 22 mounted on the housing 13. On the front of the housing, the switches and indicators are placed and protected by a fully covering foil. The pictograms for cleaning or servicing thereby show where the switches 16, 17 are located. The equipment is initiated by pressing the switch 16 for a certain duration, typically a few seconds. This takes place to avoid unintended initiation of the equipment, which possibly could happen if not authorized persons activates the switch briefly. When the equipment is initiated, the cleaning indication panel 23, which consists of a row of light-emitting diodes, will return to the initial position and one indicator is switched-on. At the same time, a timer process starts, which expires when the bed needs cleaning. Proportional to the timer process, more and more indicators 23 are switched on, for then eventually at the expiration of the timer process, having all indicators switched-on. Alternatively, one switched-on light-emitting diode indicates status for the process by its position in the row.

The equipment is also furnished with a thermo sensor 18, located directly against the front of the housing 13, for measuring the surrounding temperature of the bed. An increase in temperature can then indicate that the bed is in a washer system 2 and is being cleaned. The signal is also used for resetting the equipment so that a new timer/counter period can start. The number of cleanings is summed up by a counter for indicating a need for service and is indicated on the service indication panel 24, which consists of a row of light-emitting diodes. When a preset number of cleanings has been carried out, an indication of the need for service will appear. The need for service can be accelerated by use of stronger cleaning agents, so in order to register this, a pH-sensor 20 is connected to the housing. It is possible to connect an extern pH-sensor or to build a pH-sensor 20 directly into the housing. In the drawing, a possibility for the positioning is shown, but other positions can also be anticipated. Input from the pH-sensor during washing will be used to attribute the counter for the service indicator a factor, so a more thorough cleaning means that the counter reaches the threshold where a need for service is indicated earlier.

The indication of the need for service is reset in that a magnetic object is brought within reach of a Hall-sensor 19 located directly against the front of the housing 13. It is practical that the resetting besides from the activation of the Hall-sensor 19 with a magnet involves a pressing of the keys 17 or a combination of keys so that unintended resetting is avoided.

The cable 22 partly comprises cords for power supply but also a connection to a potential extern initiation switch located on the bed 1. If the bed 1 is cleaned in an automatic washer system 2 for beds, the washer system may be equipped with an arrangement, which activates the initiation switch, which can be a conventional switch or a switch based on a sensor, e.g. a magnetic sensor or e.g. RFID which allows duplex communication.

In the cable 22 there is also an interface for the control 15 of the bed. In this way there is access to other parameters in the use of the bed, which can be used for determining if the bed 1 needs cleaning. E.g. the number of activations of the hand control 12 is used as a supplementary input, which can trigger a request for cleaning before the expiration of the timer process. The signal, which is transferred to the control 15, is sophisticated so to understand, that the requirement for cleaning can be attributed to the individual elements in the bed: the bed frame with adjustable framework 4,5,10,11, control 15, electric actuators 6,7,8,9 and control unit 12; the mattress of the bed; the bed linen and pillow; control units as independent units. It is therefore certain that different time periods can be programmed for indication of request for cleaning for the various elements of the bed.

The equipment is microprocessor based and can thus be programmed to an indication according to the wishes of the customer. The programming is carried out from a PC with specially manufactured software connected to the control via a cable. The cable is connected to a bus-connection on the microprocessor, which is lead to a plug and socket connection on the printed circuit. Because of this programmable flexibility, it will, as a part of the invention, be possible to differentiate from the standard solution in that an indicator shows the reason that the requirement for cleaning has been triggered. This can be done by directly dedicating one or more of the light-emitting diodes in the indication panel 23 to this purpose. If it is chosen to let only one indicator serve this purpose, the frequency, with which the indicator blinks or the color of this, could be used to indicate, what has triggered the requirement for cleaning. That a LED panel is described here to indicate the need for cleaning, does not exclude that another type of display can be used, e.g. a LCD. A display can also have digits like e.g. a seven segment display or a graphic display. The same flexible structure is used in the indicator panel 24 for indication of the need for service, where the appearance of each light-emitting diode can be programmed separately.

For communication between the equipment for use in connection with cleaning of articles of hospital and care furniture and the control, which manages the adjustment of the bed, the communication system "Openbus" according to WO2007/057014 to Linak is used. Openbus describes a standard for communication between the various units attached to the bed, this being hand control(s), the individual actuator, etc. It is therefore possible to communicate various parameters between the control and the individual Openbus-connected unit. The control can via a gateway be connected to network in the hospital, which can comprise a central monitoring post, operated by the nursing staff and/or staff in charge of maintenance and cleaning. With "Openbus" is provided several possibilities for communication of status of the bed including duplex communication directly with the equipment for use in connection with cleaning of articles of hospital and care furniture. It will thus be possible to transfer the indication "cleaning required" or "servicing required" with various parameters as to what needs cleaning or servicing, directly to the central monitoring. The sorting of the indication can take place so that a request for cleaning is sent directly to the cleaning staff or the hospital porter, who is in charge of collecting the bed and bringing it to cleaning. When it comes to extern companies which take care of maintenance, an indication of service or repair could e.g. be sent via the internet from the monitoring post. If the monitoring post is connected to the database containing data about registered patients, it will from the monitoring post based on the hygienic condition of the patient be possible to program the parameters of the equipment, so that a request for intensified hygiene e.g. will trigger an indication of "cleaning required" earlier than, what is otherwise standard for the equipment. When a patient is discharged, it will be possible from the monitoring post manually or automatically to externally control the equipment for use in connection with cleaning of hospital and care furniture to show "cleaning required" and/or "servicing required", and thus ensuring that the bed will be cleaned and serviced before the next patient occupies it.

## Claims

1. An equipment in combination with a piece of hospital or care furniture e.g. a hospital or care bed, a patient lifter, a treatment table, a chair, where the piece of hospital or care furniture comprises:
- at least one actuator (6,7,8,9) for adjusting the article of furniture (1),
- at least one control unit (12),
- at least one control (15) comprising a microprocessor
**characterized in that** the equipment comprises,
- a signaling device which can give a signal, when the piece of furniture (1) has been cleaned,
- a receiver for receiving the signal from the signaling device,
- a timer and/or counter unit, which based on the signal starts a process to determine when the piece of furniture again needs cleaning, and
- an indicator (23) e.g. arranged in connection with the article of furniture (1), which indicates when the piece of furniture (1) again needs cleaning.

2. Combination according to claim 1, **characteriz ed** in that the indication of when the piece of furniture (1) needs cleaning appears after expiration of a timer function with a preset period of time.

3. Combination according to claim 1, **characterize d** in that the indication of when the piece of furniture (1) again needs cleaning appears when the number of activations of the control unit (12) exceeds a predetermined number.

4. Combination according to claim 1, **characteriz ed** in that the signaling device, which signals that the article of furniture (1) has been cleaned, is a switch or a sensor e.g. a magnetic switch or RFID.

5. Combination according to claim 4, **characteriz ed** in that the switch or sensor is activated manually by operation or automatically when the piece of furniture (1) passes an activator or signaling device, which is typically located in the washer system (2).

6. Combination according to claim 1, **characteriz ed** in that the signaling device, giving off a signal that the piece of furniture (1) has been cleaned, is a temperature sensor (18) in the equipment which gives a signal when the temperature of the equipment exceeds a threshold value.

7. Combination according to claim 1, **characteriz ed** in that the indication that the piece of furniture (1) needs to be cleaned manually can be controlled externally, e.g. by means of a switch on the piece of furniture (1), on a housing or on the control unit (12).

8. Combination according to claim 1, **characteriz ed** in that the indication that the piece of furniture (1) needs to be cleaned has been visualized by means of indicators (23) typically constructed as a LED panel, e.g. located in a housing mounted on the piece of furniture or in the control unit (12).

9. Combination according to claim 1, **characteriz ed** in that the indication that the piece of furniture (1) needs to be cleaned at the same times shows which situation has caused the triggering of the indication and the extent of the desired cleaning, e.g. by differentiating the color or blink rate of the indicator.

10. Combination according to claim 1, **characterized in that** the equipment (13) either is an independent unit or integral with a monitoring unit and the control (15) managing the adjustment of the piece of furniture.

11. Combination according to claim 1, **characterized in that** the equipment (13) is furnished with means for maintaining status and set-up when the mains supply is cut off **in that** the equipment is furnished with at least one of the following arrangements:
a. battery backup,
b. memory circuit which retains data in a no-current state,
c. minimizing of power consumption and
d. real-time clock input for reconstructing lost data.

12. Combination according to claim 1, **characterized in that** the equipment (13) is furnished with a counter unit, counting the number of times the piece of furniture (1) has been cleaned to indicate when the piece of furniture again needs servicing.

13. Combination according to claim 12, **characteried** in that the counter unit counting the number of cleanings of the piece of furniture (1) is equipped with a device for external control of the counter unit, e.g. resetting.

14. Combination according to claim 13, **characteried** in that the device for external control of the counter unit is a magnet-based switch, which is activated by placing a magnet in its presence.

15. Combination according to claim 13, **characteried** in that the device for resetting the counter unit is activated through communication with the microprocessor.

16. Combination according to claim 14, **characteried** in that the magnet-based switch is a Hall-sensor (19).

17. Combination according to claim 12, **characteried** in that the indication that the piece of furniture (1) needs to be serviced has been visualized by means of indicators (24) typically constructed as a LED panel e.g. located in a housing mounted on the piece of furniture (1) or in a control unit (12).

18. Combination according to claim 1, **characterized in that** the equipment (13) receives information concerning the pH value of the cleaning agent during washing.

19. Combination according to claim 18, **characteried** in that the equipment (13) is connected to or furnished with a measuring apparatus (20) for measuring the pH value of the cleaning agent during washing.

20. Combination according to claim 12 and 18, **characterized in that** the equipment (13) based on pH-value of the cleaning agent during washing calculates the factor which, together with the number of cleanings of the piece of furniture since the last servicing of the piece of furniture (1), forms part of the determination of when an indication of required servicing of the piece of furniture (1) appears.

21. Combination according to claim 1, **characterized in that** the equipment (13) has means for exchanging data with other units in the piece of furniture (1), where other units could be one of the following:
a. a control (15),
b. a gate way or
c. a hand control (12)
and where the data to be communicated could be:
d. the number of activations of control units,
e. status for timer or counting circuit,
f. reset or initiating of times or counters,
g. set-up timer or counter thresholds,
h. the number of cleanings since the last servicing,
i. configuration of displaying on indication panel
j. configuration of functions on keys
k. configuration of routine of calculation of pH measuring
l. external control of timers, counters or indicators.

22. Method using the equipment according to claims 1 to 21 for cleaning of hospital and care furniture, including an initiation or reset state, an operating condition with indication of the status and an alarm condition,
where the initiation state is carried out manually or automatically with a sensor, preferably a thermo sensor, resets a timer, which goes through a period of time before the next cleaning and resetting of a counter, which counts the number of operations of a control unit, such as a hand control and at the same time updates the status on an indication panel and
where the operating condition maintains and increments the timer in the process of a preset period of time and indicates the status of the process on the indication panel and at the same time maintains the counter, which counts the number of operations of the control unit, and where the alarm condition occurs when the preset time of the timer expires or the counter, which counts the number of operations of the control unit, exceeds a determined number, where the alarm condition is indicated on the indication panel.

23. Method using the equipment according to claims 1 to 21 for servicing of hospital and care furniture based on the cleaning of these, including an initiation or reset state, an operating condition with indication of the status and an alarm condition,
where the initiation state is carried out manually by activating a sensor, preferably a Hall-sensor with a magnet, and brings about a resetting of a counter, which counts the number of cleanings and at the same time updates the status on an indication panel, and
where the operating condition maintains the counter, which counts the number of cleanings of the piece of furniture and as input also has a sensor, preferably a pH sensor, which measures the aggressiveness of the cleaning agent and manipulates the counter with a factor corresponding to the extra need for service, and
where the alarm condition occurs when the counter, which counts the number of cleanings of the piece of furniture exceeds a determined number, where the alarm condition is indicated on the indication panel.

## Patentansprüche

1. Eine Ausrüstung in Kombination mit einem Krankenhaus- oder Pflege-Möbelstück, z.B. einem Krankenhaus- oder Pflegebett, einem Patientenlifter, einem Behandlungstisch, einem Stuhl, wobei das Krankenhaus- oder Pflege-Möbelstück aufweist:
- mindestens einen Aktuator (6, 7, 8, 9) zum Einstellen des Möbelstücks (1),
- mindestens eine Steuereinheit (12),
- mindestens eine Steuerung (15), die einen Mikroprozessor aufweist, **dadurch gekennzeichnet, dass** die Ausrüstung aufweist:
- eine Signalisierungseinrichtung, die ein Signal geben kann, wenn das Möbelstück (1) gereinigt worden ist,
- einen Empfänger zum Empfangen des Signals von der Signalisierungseinrichtung,
- eine Zeitgeber- und/oder Zählereinheit, die, gestützt auf das Signal, einen Prozess startet, um zu bestimmen, wann das Möbelstück erneut eine Reinigung benötigt, und
- einen Anzeiger (23), z.B. in Verbindung mit dem Möbelstück (1) angeordnet, der anzeigt, wenn das Möbelstück (1) erneut Reinigung benötigt.

2. Kombination nach Anspruch 1, **dadurch gekennzeichnet, dass** die Anzeige, wann das Möbelstück Reinigung benötigt, nach Ablauf einer Zeitgeberfunktion mit einer voreingestellten Zeitperiode erscheint.

3. Kombination nach Anspruch 1, **dadurch gekennzeichnet, dass** die Anzeige, wann das Möbelstück erneut Reinigung benötigt, erscheint, wenn die Anzahl von Aktivierungen der Steuereinheit (12) eine vorbestimmte Zahl übersteigt.

4. Kombination nach Anspruch 1, **dadurch gekennzeichnet, dass** die Signalisierungseinrichtung, die signalisiert, dass das Möbelstück (1) gereinigt worden ist, ein Schalter oder ein Sensor ist, z.B. ein Magnetschalter oder RFID.

5. Kombination nach Anspruch 4, **dadurch gekennzeichnet, dass** der Schalter oder Sensor manuell durch Betätigung oder automatisch aktiviert wird, wenn das Möbelstück (1) an einer Aktivierungs- oder Signalisierungseinrichtung vorbeiläuft, die typischerweise in dem Waschsystem (2) angeordnet ist.

6. Kombination nach Anspruch 1, **dadurch gekennzeichnet, dass** die Signalisierungseinrichtung, die ein Signal abgibt, das das Möbelstück (1) gereinigt worden ist, ein Temperatursensor (18) in der Ausrüstung ist, der ein Signal abgibt, wenn die Temperatur der Ausrüstung einen Schwellwert übersteigt.

7. Kombination nach Anspruch 1, **dadurch gekennzeichnet, dass** die Anzeige, dass das Möbelstück (1) manuell gereinigt werden soll, extern gesteuert werden kann, z.B. mit Hilfe eines Schalters auf dem Möbelstück (1), auf einem Gehäuse oder auf der Steuereinheit (12).

8. Kombination nach Anspruch 1, **dadurch gekennzeichnet, dass** die Anzeige, dass das Möbelstück (1) Reinigung benötigt, visualisiert worden ist mit Hilfe von Indikatoren (23), die typischerweise als LED-Anzeige konstruiert sind, z.B. in einem Gehäuse angeordnet sind, das auf dem Möbelstück oder in der Steuereinheit (12) montiert ist.

9. Kombination nach Anspruch 1, **dadurch gekennzeichnet, dass** die Anzeige, dass das Möbelstück (1) einer Reinigung bedarf, gleichzeitig zeigt, welche Situation das Auslösen der Anzeige und das Ausmaß der gewünschten Reinigung anzeigt, z.B. durch Differenzieren der Farbe oder Blinkrate des Indikators.

10. Kombination nach Anspruch 1, **dadurch gekennzeichnet, dass** die Ausrüstung (13) entweder eine unabhängige Einheit oder einstückig mit einer Überwachungseinheit und der Steuerung (5) ist, die die Einstellung des Möbelstücks verwaltet.

11. Kombination nach Anspruch 1, **dadurch gekennzeichnet, dass** die Ausrüstung (13) mit Mitteln ausgestattet ist zum Aufrechterhalten des Status und der Konfiguration, wenn die Netzspannungsversorgung abgeschaltet wird, wobei die Ausrüstung ausgestattet ist mit mindestens einer der folgenden Anordnungen:
a) Batterieunterstützung,
b) Speicherschaltung, die Daten in einem stromlosen Zustand aufrecht erhält,
c) Minimierung des Leistungsverbrauchs und
d) Echtzeituhreingang zum Rekonstruieren von verlorenen Daten.

12. Kombination nach Anspruch 1, **dadurch gekennzeichnet, dass** die Ausrüstung (13) ausgestattet ist mit einer Zählereinheit, die die Anzahl von Malen zählt, die das Möbelstück gereinigt worden ist, um anzuzeigen, wenn das Möbelstück erneut einer Wartung bedarf.

13. Kombination nach Anspruch 12, **dadurch gekennzeichnet, dass** die Zählereinheit, die die Anzahl von Reinigungen des Möbelstücks (1) zählt, ausgerüstet ist mit einer Einheit zum externen Steuern der Zählereinheit, z.B. Rückstellen.

14. Kombination nach Anspruch 13, **dadurch gekennzeichnet, dass** die Einrichtung für die externe Steuerung der Fehlereinheit ein Magnet-gestützter Schalter ist, der aktiviert wird, durch Anordnung eines Magneten in seiner Anwesenheit.

15. Kombination nach Anspruch 13, **dadurch gekennzeichnet, dass** die Einrichtung zum Rückstellen der Fehlereinheit aktiviert wird durch Kommunikation mit dem Mikroprozessor.

16. Kombination nach Anspruch 14, **dadurch gekennzeichnet, dass** der Magnet-gestützte Schalter ein Hall-Sensor (19) ist.

17. Kombination nach Anspruch 12, **dadurch gekennzeichnet, dass** die Anzeige, dass das Möbelstück (1) gewartet werden muss, visualisiert worden ist mit Hilfe von Indikatoren (24), die typischerweise als eine LED-Anzeige konstruiert sind, z.B. in einem Gehäuse angeordnet sind, das auf dem Möbelstück (1) oder in einer Steuereinheit (12) montiert ist.

18. Kombination nach Anspruch 1, **dadurch gekennzeichnet, dass** die Ausrüstung (13) Information empfängt bezüglich des pH-Werts der Reinigungslösung während des Waschens.

19. Kombination nach Anspruch 18, **dadurch gekennzeichnet, dass** die Ausrüstung (13) verbunden ist oder ausgerüstet ist mit einer Messvorrichtung (20) zum Messen des pH-Werts der Reinigungslösung während des Waschens.

20. Kombination nach Anspruch 12 und 18, **dadurch gekennzeichnet, dass** die Ausrüstung (13) gestützt auf den pH-Wert der Reinigungslösung während des Waschens den Faktor berechnet, der zusammen mit der Zahl der Reinigungen des Möbelstücks seit der letzten Wartung des Möbelstücks (1) einen Teil einer Bestimmung bildet, wann eine Anzeige der erforderlichen Wartung des Möbelstücks (1) erscheint.

21. Kombination nach Anspruch 1, **dadurch gekennzeichnet, dass** die Ausrüstung (13) Mittel aufweist zum Austauschen von Daten mit anderen Einheiten in dem Möbelstück (1), wobei andere Einheiten eine der Folgenden sein können:
a) eine Steuerung (15),
b) eine Schnittstelle oder
c) eine Handsteuerung (12)
und wo die zu kommunizierenden Daten sein können:
d) die Anzahl der Aktivierungen von Steuereinheiten,
e) Status für Zeitgeber- oder Zählerschaltung,
f) Rücksetzen oder Initiieren von Zeiten oder Zählern,
g) Einrichten von Zeitgeber- oder Zähler-Schwellwerten,
h) die Anzahl von Reinigungen seiL der letzten Wartung,
i) Konfiguration von Darstellungen auf Anzeigetafeln,
j) Konfiguration von Funktionen auf Tasten,
k) Konfiguration von Berechnungsroutinen von pH-Messungen,
l) externe Steuerung von Zeitgebern, Fehlern oder Indikatoren.

22. Verfahren, das die Ausrüstung nach den Ansprüchen 1 bis 21 verwendet zum Reinigen von Krankenhaus- und Pflege-Möbeln, das aufweist einen Initiierungs- oder Rücksetz-Zustand, einen Betriebszustand mit Anzeige des Status und einen Alarmzustand, wobei der Initiationszustand manuell oder automatisch mit einem Sensor, vorzugsweise einem Thermosensor, ausgeführt wird, einen Zeitgeber zurücksetzt, der durch eine Zeitperiode geht vor dem nächsten Reinigen und Rückstellen eines Zählers, der die Anzahl von Betätigungen einer Steuereinheit zählt, beispielsweise eine Handsteuerung, und zur gleichen Zeit den Status auf einer Anzeigetafel aktualisiert und
wobei der Betriebszustand den Zeitgeber aufrecht erhält und weiterzählt in dem Prozess einer voreingestellten Zeitperiode und den Status des Prozesses auf der Anzeigetafel anzeigt und zur gleichen Zeit den Zähler beibehält, der die Anzahl von Betätigungen der Steuereinheit zählt, und wo der Alarmzustand erscheint, wenn die voreingestellte Zeit des Zeitgebers ausläuft oder der Zähler, der die Anzahl von Betätigungen der Steuereinheit zählt, eine vorbestimmte Zahl übersteigt, wobei der Alarmzustand auf der Anzeigetafel angezeigt wird.

23. Verfahren, das die Ausrüstung nach Ansprüchen 1 bis 21 verwendet zum Warten von Krankenhaus- und Pflege-Möbeln gestützt auf ihre Reinigung, das aufweist einen Initiierungs- oder Rücksetz-Status, einen Befehlszustand mit Anzeige des Status und einen Alarmzustand, worin der Initiierungszustand manuell durch Aktivierung eines Sensors, vorzugsweise eines Hall-Sensors mit einem Magnet, ausgeführt wird und ein Rückstellen eines Zählers mit sich bringt, der die Zahl der Reinigungen zählt und gleichzeitig den Status auf einer Anzeigetafel aktualisiert, und worin der Betriebszustand den Zähler aufrecht erhält, der die Zahl der Reinigungen des Möbelstücks zählt, und als Eingang ebenfalls einen Sensor, vorzugsweise einen pH-Sensor aufweist, der die Aggressivität der Reinigungslösung misst und den Zähler mit einem Faktor multipliziert, der dem zusätzlichen Wartungsbedarf entspricht, und wo der Alarmzustand erscheint, wenn der Zähler, der die Anzahl von Reinigungen des Möbelstücks zählt, eine vorbestimmte Zahl übersteigt, wobei der Alarmzustand auf der Anzeigetafel angezeigt wird.

## Revendications

1. Equipement en combinaison avec un matériel d'hôpital ou un mobilier de soins, par exemple un lit d'hôpital ou un lit de soins, un élévateur de patient, une table de traitement, un fauteuil, dans lequel le matériel d'hôpital ou le mobilier de soins comprend :
- au moins un actionneur (6, 7, 8, 9) pour ajuster l'article mobilier (1),
- au moins une unité de commande (12),
- au moins une commande (15) comprenant un microprocesseur,
**caractérisé en ce que** l'équipement comprend :
- un dispositif de signalisation qui peut émettre un signal lorsque la pièce de mobilier (1) a été nettoyée,
- un récepteur pour recevoir le signal du dispositif de signalisation,
- une unité à minuterie et/ou compteur qui, sur la base du signal, lance un processus pour déterminer quand la pièce de mobilier a à nouveau besoin d'être nettoyée, et
- un indicateur (23) par exemple aménagé en liaison avec la pièce de mobilier (1), qui indique lorsque la pièce de mobilier (1) a à nouveau besoin d'être nettoyée.

2. Combinaison selon la revendication 1, **caractérisée en ce que** l'indication du moment où la pièce de mobilier (1) a à nouveau besoin d'être nettoyée apparaît après expiration d'une période de temps prédéfinie dans une fonction de minuterie.

3. Combinaison selon la revendication 1, **caractérisée en ce que** l'indication du moment où la pièce de mobilier (1) a à nouveau besoin d'être nettoyée apparaît lorsque le nombre d'activations de l'unité de commande (12) dépasse un nombre prédéterminé.

4. Combinaison selon la revendication 1, **caractérisée en ce que** le dispositif de signalisation, qui signale que l'article de mobilier (1) a été nettoyé, est un commutateur ou un capteur, par exemple, un commutateur magnétique ou un système RFID (système d'identification à distance par radiofréquences).

5. Combinaison selon la revendication 4, **caractérisée en ce que** le commutateur ou le capteur est activé manuellement par actionnement ou automatiquement lorsque la pièce de mobilier (1) passe par un activateur ou un dispositif de signalisation, qui est situé typiquement dans le système de lavage (2).

6. Combinaison selon la revendication 1, **caractérisée en ce que** le dispositif de signalisation, émettant un signal indiquant que la pièce de mobilier (1) a été nettoyée, est un capteur de température (18) dans l'équipement qui émet un signal lorsque la température de l'équipement dépasse une valeur de seuil.

7. Combinaison selon la revendication 1, **caractérisée en ce que** l'indication que la pièce de mobilier (1) a besoin d'être nettoyée manuellement peut être commandée extérieurement, par exemple au moyen d'un commutateur sur la pièce de mobilier (1), sur un boîtier ou sur l'unité de commande (12).

8. Combinaison selon la revendication 1, **caractérisée en ce que** l'indication que la pièce de mobilier (1) a besoin d'être nettoyée a été visualisée au moyen d'indicateurs (23) typiquement construits sous la forme d'un panneau de DEL, par exemple situé dans un boîtier monté sur la pièce de mobilier ou dans l'unité de commande (12).

9. Combinaison selon la revendication 1, **caractérisée en ce que** l'indication que la pièce de mobilier (1) a besoin d'être nettoyée montre en même temps la situation qui a provoqué le déclenchement de l'indication et la mesure du nettoyage souhaité, par exemple en différenciant la couleur ou la cadence de clignotement de l'indicateur.

10. Combinaison selon la revendication 1, **caractérisée en ce que** l'équipement (13) est une unité indépendante ou fait partie intégrale d'une unité de surveillance et la commande (15) gérant l'ajustement de la pièce de mobilier.

11. Combinaison selon la revendication 1, **caractérisée en ce que** l'équipement (13) est équipé de moyens pour maintenir le statut et le réglage lorsque l'alimentation du secteur est coupée en ce sens que l'équipement est équipé d'au moins l'un des aménagements suivants :
a. réserve sur batteries,
b. circuit de mémorisation qui conserve les données dans une situation sans courant,
c. minimisation de la consommation d'énergie et
d. entrée d'horloge en temps réel pour reconstruire les données perdues.

12. Combinaison selon la revendication 1, **caractérisée en ce que** l'équipement (13) est équipé d'un unité de compteur qui compte le nombre de fois que la pièce de mobilier (1) a été nettoyée pour indiquer quand la pièce de mobilier a à nouveau besoin d'être entretenue.

13. Combinaison selon la revendication 12, **caractérisée en ce que** l'unité de compteur comptant le nombre de nettoyages de la pièce de mobilier (1) est équipée d'un dispositif pour commande externe de l'unité de compteur, par exemple une remise à zéro.

14. Combinaison selon la revendication 13, **caractérisée en ce que** le dispositif pour la commande externe de l'unité de compteur est un commutateur à base d'aimant, qui est activé en plaçant un aimant en sa présence.

15. Combinaison selon la revendication 13, **caractérisée en ce que** le dispositif de remise à zéro de l'unité de compteur est activé par communication avec le microprocesseur.

16. Combinaison selon la revendication 14, **caractérisée en ce que** le commutateur à base d'aimant est un capteur de Hall (19).

17. Combinaison selon la revendication 12, **caractérisée en ce que** l'indication que la pièce de mobilier (1) a besoin d'être entretenue a été visualisée au moyens d'indicateurs (24) typiquement construits sous la forme d'un panneau de DEL, par exemple, situé dans un boîtier monté sur la pièce de mobilier (1) ou dans une unité de commande (12).

18. Combinaison selon la revendication 1, **caractérisée en ce que** l'équipement (13) reçoit des informations concernant la valeur de pH de l'agent nettoyant au cours du lavage.

19. Combinaison selon la revendication 18, **caractérisée en ce que** l'équipement (13) est connecté à un appareil de mesure (20) ou équipé de celui-ci pour mesurer la valeur de pH de l'agent nettoyant au cours du lavage.

20. Combinaison selon les revendications 12 et 18, **caractérisée en ce que** l'équipement (13) basé sur la valeur de pH de l'agent nettoyant au cours du lavage calcule le facteur qui, conjointement avec le nombre de nettoyages de la pièce de mobilier depuis le dernier entretien de la pièce de mobilier (1), fait partie de la détermination du moment où une indication d'entretien requis de la pièce de mobilier (1) apparaît.

21. Combinaison selon la revendication 1, **caractérisée en ce que** l'équipement (13) possède des moyens pour échanger des données avec d'autres unités dans la pièce de mobilier (1), dans laquelle les autres unités pourraient être l'une des suivantes :
a. une commande (15),
b. une passerelle ou
c. une commande manuelle (12),
et dans laquelle les données à communiquer pourraient être :
d. le nombre d'activations d'unités de commande,
e. le statut de la minuterie ou du circuit de comptage,
f. la remise à zéro ou l'initiation de minuteries ou de compteurs,
g. le réglage de seuils de minuteries ou de compteurs,
h. le nombre de nettoyages depuis le dernier entretien,
i. la configuration d'affichage sur un panneau d'indication,
j. les configurations de fonctions sur des touches,
k. la configuration du programme de calcul pour la mesure du pH, et
l. la commande externe de minuteries, compteurs ou indicateurs.

22. Procédé d'utilisation de l'équipement selon les revendications 1 à 21 pour le nettoyage de mobiliers d'hôpital et de soins, comprenant un état d'initiation ou de remise à zéro, une condition de fonctionnement avec indication du statut et une condition d'alarme,
dans lequel l'état d'initiation est assuré manuellement ou automatiquement avec un capteur, de préférence un capteur thermique, remet à zéro une minuterie, qui traverse une certaine période de temps avant the prochain nettoyage et la remise à zéro d'un compteur, qui compte le nombre d'opérations d'une unité de commande, telle qu'une commande manuelle, et, en même temps, met à jour le statut sur un panneau d'indication, et
dans lequel la condition de fonctionnement maintient et incrémente la minuterie dans le processus d'une période de temps prédéfinie et indique le statut du processus sur la panneau d'indication et, en même temps, maintient le compteur, qui compte le nombre d'opérations de l'unité de commande, et dans lequel la condition d'alarme se produit lorsque le temps prédéfini de la minuterie expire ou que le compteur, qui compte le nombre d'opérations de l'unité de commande, dépasse un nombre déterminé, la condition d'alarme étant indiquée sur le panneau d'indication.

23. Procédé utilisant l'équipement selon les revendications 1 à 21 pour entretenir des mobiliers d'hôpital et de soins sur la base du nettoyage de ceux-ci, comprenant un état d'initiation ou de remise à zéro, une condition de fonctionnement avec indication du statut et une condition d'alarme,
dans lequel l'état d'initiation est effectué manuellement en activant un capteur, de préférence un capteur de Hall avec un aimant et présente une remise à zéro d'un compteur qui compte le nombre de nettoyages et, en même temps, met à jour le statut sur un panneau d'indication, et
dans lequel la condition de fonctionnement maintient le compteur, qui compte le nombre de nettoyages de la pièce de mobilier, et, comme entrée, a également un capteur, de préférence un capteur de pH, qui mesure l'agressivité de l'agent nettoyant et manipule le compteur avec un facteur correspondant à un besoin supplémentaire pour l'entretien, et
dans lequel la condition d'alarme se produit lorsque le compteur, qui compte le nombre de nettoyages de la pièce de mobilier dépasse un nombre déterminé, la condition d'alarme étant indiquée sur la panneau d'indication.
